(19) Europäisches Patentamt
European Patent Office
Office européen des brevets

(11) EP 4 691 455 A1

(12) EUROPEAN PATENT APPLICATION
published in accordance with Art. 153(4) EPC

(43) Date of publication:
**11.02.2026 Bulletin 2026/07**

(21) Application number: **24779740.0**

(22) Date of filing: **19.03.2024**

(51) International Patent Classification (IPC):
*A61K 8/81* (2006.01) *A61K 8/44* (2006.01) *A61K 8/46* (2006.01) *A61K 8/73* (2006.01) *A61Q 5/02* (2006.01)

(52) Cooperative Patent Classification (CPC):
**A61K 8/44; A61K 8/46; A61K 8/73; A61K 8/81; A61Q 5/02**

(86) International application number:
**PCT/JP2024/010737**

(87) International publication number:
**WO 2024/203615 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.03.2023 JP 2023048275**

(71) Applicant: **Sumitomo Seika Chemicals Co., Ltd.**
**Kako-gun, Hyogo 675-0145 (JP)**

(72) Inventors:
- **SASAKI, Sota**
**Himeji-shi, Hyogo 672-8076 (JP)**
- **TAGUCHI, Miku**
**Himeji-shi, Hyogo 672-8076 (JP)**
- **NAKAGAWA, Machiko**
**Himeji-shi, Hyogo 672-8076 (JP)**

(74) Representative: **Vossius & Partner Patentanwälte Rechtsanwälte mbB**
**Siebertstrasse 3**
**81675 München (DE)**

# (54) POLYMER-CONTAINING COMPOSITION

(57) The present disclosure provides a water-based composition that exhibits excellent coacervate-forming ability. Specifically, the present disclosure provides a composition comprising:
(a) an anionic surfactant, 5 to 50 mass%;
(b) a cationic polymer, 0.025 to 5 mass%;
(c) a polymer containing a (meth)acrylic acid-derived structural unit, 0.1 to 5 mass%; and
(d) water, 20 to 80 mass%,
wherein the polymer (c) is defined as follows:
(A): being at least one polymer selected from the group consisting of (A-1) and (A-2) below:
(A-1) a copolymer obtained by polymerization of the following (i) and (ii); and
(A-2) a copolymer obtained by polymerization of the following (i), (ii), and (iii):
(i) (meth)acrylic acid, 100 parts by mass;
(ii) a compound having two or more ethylenically unsaturated groups, 1 part by mass or less; and
(iii) a (meth)acrylic acid alkyl ester in which the alkyl group has 10 to 30 carbon atoms, 5 parts by mass or less; and

(B): having a storage modulus (G') of 10 to 1000 Pa at a frequency dispersion of 1 rad/s as measured under the following conditions:
measurement conditions: for a neutralized aqueous solution having a degree of neutralization of 70% and containing 0.5% mass% of the polymer (c) based on the total amount of the neutralized aqueous solution, the storage modulus (G') at an angular frequency of 1 rad/s is determined based on frequency dispersion obtained by changing the angular frequency from 0.1 to 300 rad/s under conditions of a liquid temperature of 25°C and a strain of 0.1% or 1% in strain dispersion with a rheometer frequency set to 1 Hz.

EP 4 691 455 A1

## Description

Technical Field

The present disclosure relates to a composition containing a polymer, its use, and the like.

Background Art

Hair-washing compositions may contain anionic surfactants and cationic polymers as cleansing components. Presumably, coacervates formed by these components play the role of trapping silicone and oil ingredients and allowing them to be adsorbed onto hair, whereby a high degree of conditioning effect is obtained. From this viewpoint, a composition for use in washing hair is preferably a composition with high coacervate-forming ability (in other words, high coacervate yield).

Citation List

Patent Literature

PTL 1: WO2008/129493
PTL 2: JP2015-224197A
PTL 3: WO2003/094875

Summary of Invention

Technical Problem

The present inventors conducted research to provide a novel composition with high coacervate-forming ability.

Solution to Problem

The present disclosure includes, for example, the subject matter described in the following items.

Item 1.

A composition comprising:

(a) an anionic surfactant, 5 to 50 mass%;
(b) a cationic polymer, 0.025 to 5 mass%;
(c) a polymer containing a (meth)acrylic acid-derived structural unit, 0.1 to 5 mass%; and
(d) water, 20 to 80 mass%,

wherein the polymer (c) is defined as follows:

(A): being at least one polymer selected from the group consisting of (A-1) and (A-2) below:

(A-1) a copolymer obtained by polymerization of the following (i) and (ii); and
(A-2) a copolymer obtained by polymerization of the following (i), (ii), and (iii):

(i) (meth)acrylic acid, 100 parts by mass;
(ii) a compound having two or more ethylenically unsaturated groups, 1 part by mass or less; and
(iii) a (meth)acrylic acid alkyl ester in which the alkyl group has 10 to 30 carbon atoms, 5 parts by mass or less; and

(B): having a storage modulus (G') of 10 to 1000 Pa at a frequency dispersion of 1 rad/s as measured under the following conditions:
measurement conditions: for a neutralized aqueous solution having a degree of neutralization of 70% and containing

0.5% mass% of the polymer (c) based on the total amount of the neutralized aqueous solution, the storage modulus (G') at an angular frequency of 1 rad/s is determined based on frequency dispersion obtained by changing the angular frequency from 0.1 to 300 rad/s under conditions of a liquid temperature of 25°C and a strain of 0.1% or 1% in strain dispersion with a rheometer frequency set to 1 Hz.

Item 2.

The composition according to Item 1, wherein the composition has a coacervate formation rate (mass%) of 2 mass% or more as calculated using the following formula:

$$\text{Coacervate formation rate (mass \%)} = \frac{(42 - \text{supernatant (g)})}{42} \times 100$$

when 42 g of a diluted composition obtained by diluting the composition 7-fold with water is left in a constant-temperature bath at 40°C for 16 hours and centrifuged at 3000 rpm for 30 minutes to remove the supernatant so as to collect the residue as coacervates.

Item 3.

The composition according to Item 1 or 2, wherein the composition has a coacervate size of 0.1 to 30 μm when the coacervate size is determined as follows: a diluted composition obtained by diluting the composition 7-fold with water is added to a batch cell within one minute from addition of water to the composition for dilution, the batch cell is set in a laser diffraction particle size analyzer with a stirring plate set in operation and with a refractive index set to 1.60-0.10i, and the median diameter of the particle size distribution measured 50 to 100 seconds after setting the batch cell is determined as the coacervate size.

Item 4.

The composition according to any one of Items 1 to 3, wherein the anionic surfactant (a) is at least one selected from the group consisting of lauryl sulfate, laureth sulfate, lauryl monoglyceride sulfate, lauryl sarcosine or a salt thereof, lauroyl sarcosine or a salt thereof, cocoyl sulfate, cocoyl glutamate, cocoyl glycinate salt, cocoyl methylalaninate salt, cocoyl aspartate, cocoyl sarcosinate salt, tridecylbenzenesulfonate, dodecylbenzenesulfonic acid or a salt thereof, and cocoyl isethionic acid or a salt thereof.

Item 5.

The composition according to any one of Items 1 to 4, wherein the cationic polymer (b) is at least one selected from the group consisting of

a copolymer of methylvinylimidazolinium chloride and vinylpyrrolidone,
a copolymer of 1-vinyl-2-pyrrolidone and dimethylaminoethyl methacrylate,
a polymer of dimethyldiallylammonium chloride,
a copolymer of dimethyldiallylammonium chloride and acrylamide,
a copolymer of dimethyldiallylammonium chloride and acrylic acid,
a copolymer of acrylic acid, diallyldimethylammonium chloride, and acrylamide,
a copolymer of acrylic acid, methyl acrylate, and methacrylamidopropyltrimethylammonium chloride,
a copolymer of acrylamide, dimethyldiallylammonium chloride, and methacrylamidopropyltrimonium chloride,
a polymer obtained by addition of glycidyltrimethylammonium chloride to hydroxyethyl cellulose, and
a polymer obtained by addition polymerization of glycidyllauryldimethylammonium chloride to hydroxyethyl cellulose.

Item 6.

The composition according to any one of Items 1 to 5, which is for washing hair.

Item 7.

A method for washing hair using the hair-washing composition according to Item 6.

Item 8.

A method for washing hair, comprising:

applying the hair-washing composition according to Item 6 to hair; and
rinsing the applied hair-washing composition with water.

Item 9.

A method for improving the yield of coacervates by using (c) defined below, the coacervates being generated when a composition comprising the following (a) to (d) is diluted with water:

(a) an anionic surfactant, 5 to 50 mass%;
(b) a cationic polymer, 0.025 to 5 mass%;
(c) a polymer containing a (meth)acrylic acid-derived structural unit, 0.1 to 5 mass%; and
(d) water, 20 to 80 mass%,

wherein the polymer (c) is defined as follows:

(A): being at least one polymer selected from the group consisting of (A-1) and (A-2) below:

(A-1) a copolymer obtained by polymerization of the following (i) and (ii); and
(A-2) a copolymer obtained by polymerization of the following (i), (ii), and (iii):

(i) (meth)acrylic acid, 100 parts by mass;
(ii) a compound having two or more ethylenically unsaturated groups, 1 part by mass or less; and
(iii) a (meth)acrylic acid alkyl ester in which the alkyl group has 10 to 30 carbon atoms, 5 parts by mass or less; and

(B): having a storage modulus (G') of 10 to 1000 Pa at a frequency dispersion of 1 rad/s as measured under the following conditions:
measurement conditions: for a neutralized aqueous solution having a degree of neutralization of 70% and containing 0.5% mass% of the polymer (c) based on the total amount of the neutralized aqueous solution, the storage modulus (G') at an angular frequency of 1 rad/s is determined based on frequency dispersion obtained by changing the angular frequency from 0.1 to 300 rad/s under conditions of a liquid temperature of 25°C and a strain of 0.1% or 1% in strain dispersion with a rheometer frequency set to 1 Hz.

Item 10.

The method for improving the yield of coacervates according to Item 9,

wherein the coacervate formation rate (mass%) is 2 mass% or more as calculated using the following formula:

$$\text{Coacervate formation rate (mass \%)} = \frac{(42 - \text{supernatant (g)})}{42} \times 100$$

when 42 g of a diluted composition obtained by diluting the composition comprising (a) to (d) 7-fold with water is left in a constant-temperature bath at 40°C for 16 hours and centrifuged at 3000 rpm for 30 minutes to remove the supernatant so as to collect the residue as coacervates.

Item 11.

A method for improving the yield of coacervates, comprising diluting the composition according to any one of Items 1 to 6 with water to generate coacervates,
wherein the yield of coacervates is improved as compared to when coacervates are generated by diluting with water a composition comprising the components (a) to (d), the component (c) being 0.1 to 5 mass% of a polymer containing a (meth)acrylic acid-derived structural unit that does not satisfy the condition (B).

Advantageous Effects of Invention

The present disclosure provides a novel composition that exhibits excellent coacervate-forming ability. The composition is particularly useful as a hair-washing composition.

Brief Description of Drawings

Fig. 1 shows the strain distribution of a neutralized aqueous solution having a degree of neutralization of 70% and containing 0.5 mass% of a polymer (component (c)) based on the total amount of the neutralized aqueous solution, at a liquid temperature of 25°C and a rheometer frequency of 1 Hz. The horizontal axis represents the percentage of strain, and the vertical axis represents the storage modulus (G'). The figure shows an outline for determining whether to use a strain value of 0.1% or 1% as a condition for measuring the G' value (storage modulus) of the polymer (component (c)) at a frequency dispersion of 1 rad/s (this figure is a conceptual diagram unrelated to the measurement results of samples used in the examples). This diagram shows that when the linear region obtained in the strain distribution measurement at 1 Hz includes 1% strain, 1% is selected as the strain value; whereas when the linear region includes 0.1% strain but does not include 1%, 0.1% is selected as the strain value.

Fig. 2 shows the frequency dispersion of a neutralized aqueous solution having a degree of neutralization of 70% and containing 0.5 mass% of a polymer (component (c)) based on the total amount of the neutralized aqueous solution. The frequency dispersion is obtained by changing the angular frequency of the neutralized aqueous solution from 0.1 to 300 rad/s with a strain of 0.1% or 1% determined at a liquid temperature of 25°C and a rheometer frequency of 1 Hz. The horizontal axis represents the angular frequency, and the vertical axis represents the storage modulus (G'). This figure is a conceptual diagram unrelated to the measurement results of samples used in the examples.

Description of Embodiments

The following describes embodiments included in the present disclosure in more detail. The present disclosure preferably encompasses, but is not limited to, a polymer-containing composition, its use, its production method, and the like. The present disclosure encompasses all that is disclosed in the present specification and that would be recognized by a person skilled in the art.

The polymer-containing composition encompassed by the present disclosure contains (a) an anionic surfactant, (b) a cationic polymer, (c) a specific polymer containing a (meth)acrylic acid-derived structural unit, and (d) water. Herein, these components are sometimes referred to as "the components (a) to (d)" and the polymer-containing composition is sometimes referred to as "the composition of the present disclosure." The component (c) is a polymer having a carboxy group in the polymer chain and is thus an anionic polymer; it is not a surfactant. Thus, the component (c) is distinguished from the component (a) and the component (b).

The component (a) (anionic surfactant) may preferably be a publicly known anionic surfactant generally used in hair-washing compositions.

Examples include higher alkyl sulfate ester salts, such as sodium lauryl sulfate and potassium lauryl sulfate; alkyl ether sulfate ester salts, such as POE-triethanolamine lauryl sulfate and sodium POE-lauryl sulfate; amino acid-based anionic surfactants, such as cocoyl glutamic acid triethanolamine and potassium cocoyl glycinate; N-acyl sarcosinic acids, such as sodium lauroyl sarcosinate; higher fatty acid amidosulfonates, such as sodium N-myristoyl-N-methyl taurate, sodium cocoyl methyl taurate, and sodium lauryl methyl taurate; phosphate ester salts, such as sodium POE-oleyl ether phosphate and POE-stearyl ether phosphate; sulfosuccinates, such as sodium di-2-ethylhexyl sulfosuccinate, sodium monolauroyl monoethanolamide polyoxyethylene sulfosuccinate, and sodium lauryl polypropylene glycol sulfosuccinate; alkylbenzene sulfonates, such as sodium linear dodecylbenzenesulfonate, triethanolammonium linear dodecylbenzene sulfonate, and dodecylbenzenesulfonic acid; N-acyl glutamates, such as monosodium N-lauroyl glutamate, disodium N-stearoyl glutamate, and monosodium N-myristoyl-L-glutamate; higher fatty acid ester sulfate salts, such as sodium hydrogenated coconut fatty acid glycerol sulfate; POE-alkyl ether carboxylic acids; POE-alkyl allyl ether carboxylates; $\alpha$-olefin sulfonates; higher fatty acid ester sulfonates; secondary alcohol sulfate ester salts; higher fatty acid alkylolamide sulfate ester salts; sodium lauroyl monoethanolamide succinate; N-palmitoyl aspartic acid ditriethanolamine; and sodium caseinate.

More specific examples of these include lauryl sulfate, laureth sulfate, lauryl monoglyceride sulfate, lauryl sarcosine or a salt thereof, lauroyl sarcosine or a salt thereof, cocoyl or a salt thereof (more specifically, cocoyl sulfate, cocoyl glutamate, cocoyl glycinate salt, cocoyl methylalaninate salt, cocoyl aspartate, and cocoyl sarcosinate salt), tridecylbenzenesulfonate, dodecylbenzenesulfonic acid or a salt thereof, and cocoyl isethionic acid or a salt thereof. Examples of these salts include alkali metal salts (in particular, sodium salts and potassium salts), ammonium salts,

triethylamine salts, and mono-, di-, or triethanolamine salts.

The component (a) can be used singly or in a combination of two or more.

The composition of the present disclosure contains 5 to 50 mass% of the component (a). The upper limit and the lower limit of the range may be, for example, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49 mass%. For example, the range may be from 10 to 40 mass%.

The component (b) (cationic polymer) may preferably be a publicly known cationic polymer generally used in hair-washing compositions. Examples include those having cationic nitrogen-containing groups, such as quaternary ammonium, protonated amino groups, and mixtures thereof.

More specific examples of the component (b) include copolymers of vinyl monomers having cationic amine or quaternary ammonium functionality and at least one selected from the group consisting of (meth)acrylamide, alkyl or dialkyl (meth)acrylamides, alkyl (meth)acrylates, vinyl caprolactam, and vinylpyrrolidone. The alkyl- or dialkyl-substituted monomer preferably has a C1-C7 alkyl group, more preferably a C1-C3 alkyl group. Even more specific examples include copolymers of vinyl monomers having cationic amine quaternary ammonium functionality and at least one selected from the group consisting of vinyl ester, vinyl alcohol, maleic anhydride, propylene glycol, and ethylene glycol.

The cationic amine may be a primary, secondary, or tertiary amine, preferably a secondary or tertiary amine, and more preferably a tertiary amine.

Preferred examples of the component (b) include polymeric quaternary ammonium salts. More specific examples include the following polymeric quaternary ammonium salts:

a copolymer of methylvinylimidazolinium chloride and vinylpyrrolidone (polyquaternium-16);
a copolymer of 1-vinyl-2-pyrrolidone and dimethylaminoethyl methacrylate (polyquaternium-11);
a polymer of dimethyldiallylammonium chloride (polyquaternium-6);
a copolymer of dimethyldiallylammonium chloride and acrylamide (polyquaternium-7);
a copolymer of dimethyldiallylammonium chloride and acrylic acid (polyquaternium-22);
a copolymer of acrylic acid, diallyldimethylammonium chloride, and acrylamide (polyquaternium-39);
a copolymer of acrylic acid, methyl acrylate, and methacrylamidopropyltrimethylammonium chloride (polyquaternium-47); and
a copolymer of acrylamide, dimethyldiallylammonium chloride, and methacrylamidopropyltrimonium chloride (polyquaternium-94).

Examples of the component (b) also include cationic polysaccharide polymers, such as cationic cellulose derivatives, cationic starch derivatives, and cationic guar gum derivatives. Such cationic polysaccharide polymers having a charge density of 0.1 to 4 meq/g are more preferred.

Preferred examples of cationic cellulose derivatives include a polymeric quaternary ammonium salt (polyquaternium-10) obtained by addition of glycidyltrimethylammonium chloride to hydroxyethylcellulose and a polymeric quaternary ammonium salt (polyquaternium-24) obtained by addition polymerization of glycidyllauryldimethylammonium chloride to hydroxyethylcellulose.

Examples of cationic starch derivatives include quaternary nitrogen-containing cellulose ethers and copolymers of etherified cellulose and starch.

Examples of cationic guar gum derivatives include guar hydroxypropyltrimonium chloride.

The component (b) can be used singly or in a combination of two or more.

The composition of the present disclosure contains 0.025 to 5 mass% of the component (b). The upper limit and the lower limit of the range may be, for example, 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, or 4.9 mass%. For example, the range may be from 0.1 to 3 mass%.

The component (c) (a specific polymer containing a (meth)acrylic acid-derived structural unit) is (A), i.e., at least one polymer selected from the group consisting of (A-1) and (A-2) below:

(A-1) a copolymer obtained by polymerization of the following (i) and (ii); and
(A-2) a copolymer obtained by polymerization of the following (i), (ii), and (iii):

(i) (meth)acrylic acid, 100 parts by mass;
(ii) a compound having two or more ethylenically unsaturated groups, 1 part by mass or less; and
(iii) a (meth)acrylic acid alkyl ester in which the alkyl group has 10 to 30 carbon atoms, 5 parts by mass or less.

The term "(meth)acrylic acid" as used herein can be rephrased as "methacrylic acid and/or acrylic acid."

The component (c) can be used singly or in a combination of two or more.

The copolymer (A-1) and the copolymer (A-2) are sometimes referred to as "the polymer (A-1)" and "the polymer (A-2)," respectively. The component (c) includes the polymer (A-1) and the polymer (A-2) and is a water-soluble polymer having carboxy groups. Unless otherwise specified, the water-soluble polymer having carboxy groups also encompasses a compound in which the carboxy groups in the polymer are not neutralized (an unneutralized compound) and a compound in which some or all of the carboxyl groups in the polymer are neutralized (a neutralized compound).

Herein, the polymer (A-1) and the polymer (A-2) are concepts that encompass compounds in which (meth)acrylic acid and a compound having two or more ethylenically unsaturated groups (crosslinking agent) are polymerized (i.e., compounds in which (meth)acrylic acid is crosslinked with a compound having two or more ethylenically unsaturated groups). Thus, the polymer (A-1) and the polymer (A-2) encompass compounds in which a compound having two or more ethylenically unsaturated groups is also polymerized in addition to (meth)acrylic acid (i.e., a compound in which a (meth) acrylic acid polymer is crosslinked with the compound).

The amount of (ii) for use is 1 part by mass or less, preferably 0.01 to 1 part by mass, per 100 parts by mass of (meth)acrylic acid. The upper limit and the lower limit of the range (0.01 to 1 part by mass) maybe, for example, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, or 0.95 parts by mass. For example, the range is more preferably from 0.05 to 0.9 parts by mass, and more preferably from 0.1 to 0.7 parts by mass.

The amount of (iii) for use is 5 parts by mass or less, preferably 0.01 to 5 parts by mass, per 100 parts by mass of (meth)acrylic acid. The upper limit and the lower limit of the range (0.01 to 5 parts by mass) may be, for example, 0.05, 0.1, 0.2, 0.5, 1, 1.5, 2, 2.5, 3, 3.5, 4, or 4.5 parts by mass. For example, the range is more preferably from 0.05 to 4 parts by mass.

Examples of (ii) include pentaerythritol tetraallyl ether, N,N'-methylenebisacrylamide, ethylene glycol dimethacrylate, ethylene glycol diglycidyl ether, polyethylene glycol dimethacrylate, and polyethylene glycol diglycidyl ether. As described above, (ii) is a compound that can act as a crosslinking agent. The compound (ii) can be used singly or in a combination of two or more.

(iii) is an ester of (meth)acrylic acid and a higher alcohol in which the alkyl group has 10 to 30 carbon atoms. More preferably, the carbon number of the alkyl group is, for example, 12 to 30, 14 to 28, 16 to 26, or 18 to 24. Examples of these (meth)acrylic acid alkyl esters include stearyl methacrylate, eicosanyl methacrylate, behenyl methacrylate, and tetracosanyl methacrylate. These (meth)acrylic acid alkyl esters may be used singly or in a combination of two or more. Commercial products, such as Blemmer VMA70 (trade name, available from NOF Corporation), may also be used as such (meth)acrylic acid alkyl esters.

As described above, the component (c) is a polymer containing a (meth)acrylic acid-derived structural unit, and is thus a polymer that can be obtained using a (meth)acrylic acid as a monomer. The polymers (A-1) and (A-2) are obtained by polymerizing at least (meth)acrylic acid and a compound having two or more ethylenically unsaturated groups. A preferred method for polymerizing (i) and (ii), or (i), (ii) and (iii), is to polymerize them in a polymerization solvent in the presence of a radical polymerization initiator. A more detailed description is given below.

Examples of radical polymerization initiators include $\alpha,\alpha'$-azobisisobutyronitrile, 2,2'-azobis-2,4-dimethylvaleronitrile, 2,2'-azobismethylisobutyrate, 2,2'-azobis(2-methylpropionamidine) dihydrochloride, benzoyl peroxide, lauroyl peroxide, cumene hydroperoxide, and tertiary butyl hydroperoxide. These radical polymerization initiators can be used singly or in a combination of two or more.

The amount of the radical polymerization initiator for use is preferably 0.01 to 0.45 parts by mass, and more preferably 0.01 to 0.4 parts by mass, per 100 parts by mass of (meth)acrylic acid.

The polymers (A-1) and (A-2) both can be suitably prepared by precipitation polymerization or reversed-phase suspension polymerization, for example. Additionally, precipitation polymerization may be performed in a polymerization solvent containing a nonionic surfactant having a polyoxyethylene chain.

Preferred examples of the nonionic surfactant having a polyoxyethylene chain include ethylene oxide adducts of a polyhydric alcohol fatty acid ester, block copolymers of hydroxy fatty acid and ethylene oxide, and polyoxyethylene castor oil.

Preferred examples of ethylene oxide adducts of a polyhydric alcohol fatty acid ester include ester compounds of polyoxyethylene hydrogenated castor oil and polyhydric alcohol fatty acid. The polyhydric alcohol fatty acid is preferably a saturated or unsaturated polyhydric (particularly dihydric) alcohol fatty acid having 14 to 24 carbon atoms. More specifically, isopalmitic acid, isostearic acid, isooleic acid, and the like are preferred. In the ester compounds of polyoxyethylene hydrogenated castor oil and polyhydric alcohol fatty acid, the average number of moles of ethylene oxide added to form polyoxyethylene may be about 20 to 100 or about 30 to 70. A particularly preferred ester compound of polyoxyethylene hydrogenated castor oil and polyhydric alcohol fatty acid is polyoxyethylene hydrogenated castor oil isostearate.

The polyoxyethylene castor oil is preferably one in which the number of moles of ethylene oxides added is about 2 to 10, and more preferably one in which the number of moles of ethylene oxides added is about 2 to 5.

The block copolymers of hydroxy fatty acid and ethylene oxide can also be rephrased as "copolymers of polyhydroxy fatty acid and polyoxyethylene." The fatty acid of polyhydroxy fatty acid is preferably a fatty acid having about 14 to 22 carbon atoms, preferably myristic acid, palmitic acid, or stearic acid. The hydroxy fatty acid is preferably

hydroxymyristic acid, hydroxypalmitic acid, or hydroxystearic acid, and particularly preferably hydroxystearic acid. The hydroxystearic acid is particularly preferably 12-hydroxystearic acid. The polyhydroxy fatty acid is particularly preferably polyhydroxystearic acid. A particularly preferred block copolymer of hydroxy fatty acid and ethylene oxide is a block copolymer of 12-hydroxystearic acid and ethylene oxide.

The nonionic surfactant having a polyoxyethylene chain can be used singly or in a combination of two or more.

The amount of the nonionic surfactant having a polyoxyethylene chain for use is preferably 0.5 to 10 parts by mass and more preferably 1 to 7.5 parts by mass per 100 parts by mass of (meth)acrylic acid.

The polymerization solvent is preferably a solvent that dissolves (i), (ii), and (iii) and that does not dissolve the resulting polymer. Specific examples of such polymerization solvents include n-pentane, n-hexane, n-heptane, n-octane, isooctane, cyclopentane, methylcyclopentane, cyclohexane, methylcyclohexane, benzene, toluene, xylene, chlorobenzene, ethylene dichloride, ethyl acetate, isopropyl acetate, ethyl methyl ketone, and isobutyl methyl ketone. Of these polymerization solvents, from the standpoint of stable quality and availability, ethylene dichloride, n-hexane, n-heptane, and ethyl acetate are preferred. These polymerization solvents can be used singly or in a combination of two or more.

The amount of the polymerization solvent for use is preferably 200 to 10000 parts by mass, and more preferably 300 to 2000 parts by mass, per 100 parts by mass of (meth)acrylic acid. By using the polymerization solvent within the above range, even if the polymerization reaction proceeds, aggregation of the polymer can be suitably suppressed, allowing the polymer to be stirred uniformly and the polymerization reaction to proceed more efficiently.

The atmosphere for performing the polymerization reaction is, for example, an inert gas atmosphere, such as nitrogen gas or argon gas.

The reaction temperature for the polymerization reaction is, for example, preferably 50 to 90°C, and more preferably 55 to 75°C. When performed at these reaction temperatures, the polymerization reaction can suitably suppress the increase in viscosity of the reaction solution and further facilitate reaction control.

The reaction time for the polymerization reaction is typically 2 to 10 hours.

After completion of the reaction, the reaction solution is heated to 80 to 120°C to remove the polymerization solvent, whereby polymer in a fine powder form can be isolated.

The component (c) is a polymer having a storage modulus (G') of 10 to 1000 Pa at a frequency dispersion of 1 rad/s as measured under the following conditions:

measurement conditions: for a neutralized aqueous solution having a degree of neutralization of 70% and containing 0.5% mass% of the polymer (c) based on the total amount of the neutralized aqueous solution, the storage modulus (G') at an angular frequency of 1 rad/s is determined based on frequency dispersion obtained by changing the angular frequency from 0.1 to 300 rad/s under conditions of a liquid temperature of 25°C and a strain of 0.1% or 1% in strain dispersion with a rheometer frequency set to 1 Hz.

Satisfying the range of the storage modulus (G') is sometimes referred to as "satisfying the condition (B)."

The storage modulus (G') is determined according to the following steps.

(1) Production of neutralized aqueous solution for measurement

The component (c) is dispersed in water such that the polymer (component (c)) concentration is 0.5% based on the total amount of the neutralized aqueous solution, and an alkaline aqueous solution is added thereto under stirring to dissolve the polymer while adjusting the degree of neutralization of the polymer to 70%, thus obtaining a neutralized aqueous solution.

(2) Determination of strain based on strain distribution

The temperature of the neutralized aqueous solution prepared in (1) is set to 25°C, and the frequency of a rheometer (e.g., TA Instruments (model: AR-2000ex)) is set to 1 Hz. Subsequently, the storage modulus (G') is measured while applying strain. When the storage modulus (G') is found to be decreasing, the measurement is stopped (a graph as shown in Fig. 1 is obtained).

With reference to the obtained strain dispersion graph, the strain is determined to be 1% when the storage modulus (G') maintains a constant value up to a strain of 1% but fails to maintain a constant value up to 10%, whereas the strain is determined to be 0.1% when the storage modulus (G') maintains a constant value up to a strain of 0.1% but fails to maintain a constant value up to 1%.

(3) Determination of storage modulus based on frequency dispersion

The angular frequency of the neutralized aqueous solution prepared in (1) is changed from 0.1 to 300 rad/s with a strain of 0.1% or 1% determined based on the above criteria at a liquid temperature of 25°C (a graph as shown in Fig. 2 is obtained).

The storage modulus (G') at an angular frequency of 1 rad/s is determined based on the measurements.

The storage modulus (G') is 10 to 1000 Pa. The upper limit and the lower limit of the range may be, for example, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, or 990 Pa. For example, the range may be from 20 to 950 Pa, preferably from 100 to 500 Pa. The storage modulus (G') can be adjusted by varying the amount of a crosslinking agent added. Usually, G' is improved by increasing the amount added. More specifically, in the polymer (A), G' tends to improve when the amount of (ii) for use is increased.

The composition of the present disclosure contains 0.1 to 5 mass% of the component (c). The upper limit and the lower limit of the range may be, for example, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, or 4.9 mass%. For example, the range may be from 0.2 to 3 mass%.

The composition of the present disclosure contains 20 to 80 mass% of the component (d) (water). The upper limit and the lower limit of the range may be, for example, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, or 79 mass%. For example, the range may be from 25 to 75 mass%.

The mass ratio of the component (b) to the component (c) (i.e., (b)/(c)) is not particularly limited but is preferably about 0.2 to 10. The upper limit and the lower limit of the range may be, for example, 0.5, 1, 2, 3, 4, 5, 6, 7, 8, or 9. For example, the range may be from about 0.5 to 4.

The mass ratio of the component (a) to the component (b) (i.e., (a)/(b)) is not particularly limited but is preferably about 10 to 100. The upper limit and the lower limit of the range may be, for example, 15, 20, 25, 30, 35, 40, 45, 50, 55, 60, 65, 70, 75, 80, 85, 90, or 95. For example, the range may be from about 30 to 90, from about 40 to 80, or from about 50 to 70.

In addition to the components (a) to (d), the composition of the present disclosure can contain other components that are publicly known as additives for hair-washing compositions as long as the effect of the invention of the present disclosure is not impaired.

Examples of such other components include (e) an amphoteric surfactant, (f) a neutralizer, and (g) a conditioning agent. These components are sometimes referred to as "the components (e) to (g)."

Examples of the component (e) include alkylamine oxides, alkyl betaines, alkylamide betaines, alkyl sulfobetaines, alkylglycinate salts, alkylcarboxyglycinate salts, alkylamphopropionates, alkylamphoglycinate salts, alkylamidopropyl hydroxysultaines, acyl taurate salts, and acyl glutamates. The alkyl and acyl groups have 5 to 22 (5, 6, 7, 8, 9, 10, 11, 12, 13, 14, 15, 16, 17, 18, 19, 20, 21, or 22), preferably 7 to 19, carbon atoms.

More specifically, an alkylamide betaine represented by the following formula is preferred:

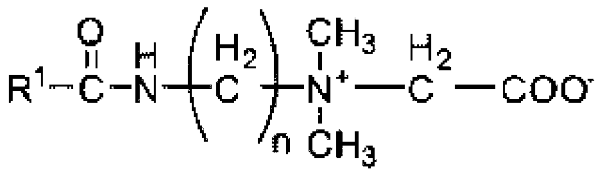

wherein $R^1$ represents a linear or branched alkyl group having 5 to 22 (5, 6, 7, 8, 9, 10, 11, 12, 13, 14, 15, 16, 17, 18, 19, 20, 21, or 22) carbon atoms. The betaine may be a mixture of those with different $R^1$s. For example, those in which $R^1CO$- is a coconut oil fatty acid residue are also preferably included. n represents 1 to 6 (1, 2, 3, 4, 5, or 6). In particular, n is preferably 3 (alkylamidopropyl betaine).

More specific examples of the betaine include coconut oil fatty acid amidopropyl betaine (i.e., cocamidopropyl betaine: CAPB) and lauric acid amidopropyl betaine.

For example, an alkyl betaine represented by the following formula is also preferred:

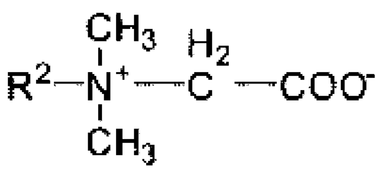

wherein $R^2$ represents a linear or branched alkyl group having 1 to 22 (1, 2, 3, 4, 5, 6, 7, 8, 9, 10, 11, 12, 13, 14, 15, 16, 17, 18, 19, 20, 21, or 22) carbon atoms.

More specific examples of the betaine include trimethylaminoacetic acid betaine (trimethylglycine), lauryl

dimethyl acetic acid betaine, and tetradecyl dimethylaminoacetic acid betaine.

The component (e) can be used singly or in a combination of two or more.

When the component (e) is used, the composition of the present disclosure may contain about 3 to 30 mass% of the component (e), for example. The upper limit and the lower limit of the range may be, for example, 4, 5, 6, 7, 8, 9, 10, 11, 12, 13, 14, 15, 16, 17, 18, 19, 20, 21, 22, 23, 24, 25, 26, 27, 28, or 29 mass%. For example, the range may be from about from 5 to 25 mass%.

When the component (e) is used, the mass ratio of the component (a) to the component (e) (i.e., (a)/(e)) is not particularly limit but is preferably about 0.2 to 5. The upper limit and the lower limit of the range may be, for example, 0.5, 1, 2, 3, or 4. For example, the range may be from about 0.5 to 4.

Preferred examples of the component (f) (neutralizer) include alkali metal hydroxides. Specifically, sodium hydroxide and potassium hydroxide are preferred.

Preferred examples also include organic amines. Specifically, triethanolamine and the like are preferred.

The component (f) can be used singly or in a combination of two or more.

When the component (f) is used, the amount for use can be suitably adjusted depending on the viscosity and the like of the composition of the present disclosure. For example, the amount may be 0.1 mol% or more per mole of the carboxy group derived from (meth)acrylic acid in the component (c). The upper limit is not particularly limited. For example, it may be about 15 mol% per mole of the carboxy group 1. The upper or lower limit of the range (0.1 to 15 mol%) may be, for example, 0.2, 0.25, 0.3, 0.4, 0.5, 0.6, 0.7, 0.8, 0.9, 1, 2, 3, 4, 5, 6, 7, 8, 9, 10, 11, 12, 13, or 14 mol%. The range may be from 0.2 to 10 mol%, for example.

The component (g) (conditioning agent) is preferably an oily component. The oily component is preferably one that can impart a moisturized feel to hair. Examples of such oily components include fats and oils, waxes, hydrocarbons, ester oils, higher alcohols, higher fatty acids, alkyl glyceryl ethers, silicones, and polyhydric alcohols.

Specific examples of oils and fats include *Argania spinosa* kernel oil, olive oil, camellia oil, shea butter, almond oil, safflower oil, sunflower oil, soybean oil, cottonseed oil, sesame oil, corn oil, rapeseed oil, rice bran oil, rice germ oil, grape seed oil, avocado oil, macadamia nut oil, castor oil, coconut oil, evening primrose oil, apricot kernel oil, persic oil, peach kernel oil, palm oil, and egg yolk oil.

Specific examples of waxes include lanolin, beeswax, candelilla wax, carnauba wax, and jojoba oil.

Specific examples of hydrocarbons include paraffin, olefin oligomers, polyisobutene, hydrogenated polyisobutene, mineral oil, squalane, polybutene, polyethylene, microcrystalline wax, petrolatum, liquid paraffin, light isoparaffin, light liquid isoparaffin, isoparaffin, $\alpha$-olefin oligomers, and synthetic squalane.

Specific examples of ester oils include diisopropyl adipate, isopropyl myristate, cetyl octanoate, isononyl isononanoate, octyldodecyl myristate, isopropyl palmitate, stearyl stearate, myristyl myristate, isotridecyl myristate, 2-ethylhexyl palmitate, octyldodecyl ricinoleate, cholesteryl/lanosteryl esters of fatty acids having 10 to 30 carbon atoms, cetyl lactate, lanolin acetate, ethylene glycol di-2-ethylhexanoate, pentaerythritol fatty acid esters, dipentaerythritol fatty acid esters, cetyl caprate, glyceryl tricaprylate, diisostearyl malate, dioctyl succinate, cetyl 2-ethylhexanoate, and hydrogenated castor oil isostearate.

Specific examples of higher alcohols include cetyl alcohol (cetanol), 2-hexyldecanol, stearyl alcohol, isostearyl alcohol, cetostearyl alcohol, oleyl alcohol, arachyl alcohol, behenyl alcohol, 2-octyldodecanol, lauryl alcohol, myristyl alcohol, decyltetradecanol, and lanolin alcohol.

Specific examples of higher fatty acids include lauric acid, myristic acid, palmitic acid, stearic acid, behenic acid, isostearic acid, 12-hydroxystearic acid, oleic acid, lanolin fatty acid, and 18-methyleicosanoic acid.

Specific examples of alkyl glyceryl ethers include batyl alcohol, chimyl alcohol, selachyl alcohol, and isostearyl glyceryl ether.

Specific examples of silicones include dimethylpolysiloxane (dimethicone), methylphenylpolysiloxane, decamethylcyclopentasiloxane, dodecamethylcyclohexasiloxane, hydroxy-terminated dimethylpolysiloxane, high-molecular-weight silicone, amino-modified silicone, betaine-modified silicone, alkyl-modified silicone, alkoxy-modified silicone, mercaptomodified silicone, carboxy-modified silicone, fluorine-modified silicone, and polyether-modified silicone. One of these oily components may be used singly, or two or more of these oily components may be used in a combination.

Examples of polyhydric alcohols include glycols and glycerol. Examples of glycols include ethylene glycol, diethylene glycol, triethylene glycol, polyethylene glycol, high-molecular-weight polyethylene glycol, propylene glycol, dipropylene glycol, polypropylene glycol, isoprene glycol, and 1,3-butylene glycol. Examples of glycerol include glycerol, diglycerol, and polyglycerol. One of these polyhydric alcohols may be used singly, or two or more of these polyhydric alcohols may be used in a combination.

The component (g) can be used singly or in a combination of two or more.

When the component (g) is used, the composition of the present disclosure may contain about 5 mass% or less of the component (g), for example. The lower limit is not particularly limited but is 0.1 mass% or more, for example. The upper limit and the lower limit of the range (0.1 to 5 mass%) may be, for example, 0.2, 0.5, 1, 1.5, 2, 2.5, 3, 3.5, 4, or 4.5 mass%. For example, the range may be from 0.2 to 4 mass%.

Preferably, the composition of the present disclosure has a pH of about 6 to 8. The pH is a value measured using a pH meter.

The composition of the present disclosure exhibits excellent coacervate-forming ability. In particular, the coacervate formation rate calculated by the following method is preferably 2 mass% or more, more preferably 2.5, 3, 3.5, or 4 mass% or more. The upper limit of the coacervate formation rate is not particularly limited but is 20 mass% or less, for example. The upper limit may be 19, 18, 17, 16, 15, 14, 13, or 12 mass% or less, for example.

**Coacervate Formation Rate Calculation Method**

42 g of a diluted composition obtained by diluting the composition comprising (a) to (d) 7-fold with water is left in a constant-temperature bath at 40°C for 16 hours and centrifuged at 3000 rpm for 30 minutes to remove the supernatant so as to collect the residue as coacervates. The coacervate formation rate (mass%) is calculated using the following formula:

$$\text{Coacervate formation rate (mass \%)} = \frac{(42 - \text{supernatant (g)})}{42} \times 100.$$

The composition of the present disclosure also exhibits a relatively small coacervate size. Since hair has many irregularities, presumably, a smaller coacervate size may provide a greater conditioning effect. More specifically, the composition of the present disclosure preferably has a coacervate size (median diameter) of 0.1 to 30 μm as measured by the following method. The upper limit and the lower limit of the range may be, for example, 0.5, 1, 2, 3, 4, 5, 6, 7, 8, 9, 10, 11, 12, 13, 14, 15, 16, 17, 18, 19, 20, 21, 22, 23, 24, 25, 26, 27, 28, or 29 μm. For example, the range is more preferably from 0.5 to 20 μm and even more preferably from 1 to 10 μm.

**Coacervate Size Measurement**

The coacervate size is determined as follows: a diluted composition obtained by diluting the composition 7-fold with water is added to a batch cell, the batch cell is set in a laser diffraction particle size analyzer with a stirring plate set in operation and with a refractive index set to 1.60-0.10i, and the median diameter of the particle size distribution measured 50 to 100 seconds after setting the batch cell is determined as the coacervate size. When diluting the composition 7-fold with water, mixing and transferring of the mixture to the batch cell is finished within one minute from addition of water to the composition.

For example, SALD-7100 (available from Shimadzu Corporation) can be used as the laser diffraction particle size analyzer.

The present disclosure also encompasses a method for improving the yield of coacervates generated when a composition containing the components (a) to (d) is diluted with water, wherein the composition contains, as the component (c), 0.1 to 5 mass% of a polymer containing a (meth)acrylic acid-derived structural unit, the polymer being the polymer (A) and satisfying the condition (B). In particular, the present disclosure preferably encompasses a method for improving the yield of coacervates, wherein the yield of coacervates is improved as compared to when coacervates are generated by diluting with water a composition comprising the components (a) to (d), the component (c) being 0.1 to 5 mass% of a polymer containing a (meth)acrylic acid-derived structural unit that does not satisfy the condition (B) (in particular, the component (c) is the polymer (A) that does not satisfy the condition (B)).

The dilution ratio with water is not particularly limited as long as the effect can be obtained, but may be, for example, about 2 to 10 times, or may be, for example, about 3 to 9 times.

In the present specification, the term "comprising" includes the concepts of consisting essentially of and consisting of. The present disclosure also encompasses any and every combination of the elements described in the present specification.

The various properties (characteristics, structures, functions, etc.) described above for each embodiment of the present disclosure may be combined in any way in identifying the subject matter encompassed by the present disclosure. Specifically, the disclosure encompasses all subject matter formed of any and every combination of the combinable properties described in the specification.

Examples

The following describes embodiments of the present disclosure in more detail with reference to Examples. However, the embodiments of the disclosure are not limited to the Examples below.

The storage modulus (G') of each of the polymers prepared in the following Production Examples 1 to 5 was measured as follows.

**Storage Modulus (G') Measurement Method**

(1) Production of neutralized aqueous solution for measurement

The polymers (1.5 g each) were separately added to 500 ml beakers each containing 288.8 g of ion-exchanged water while stirring at 500 rpm with a stirrer having four paddles with a diameter of 7 cm. After stirring for 60 minutes, 9.7 g of a 6 mass% aqueous sodium hydroxide solution was added to adjust the degree of neutralization of each polymer to 70%, and the mixture was further stirred at 300 rpm for 15 minutes, thus preparing a 0.5 mass% aqueous neutralized solution of each polymer. The pH was measured at 25°C using a pH meter (available from HORIBA, Ltd.; model number: D-51).

(2) Determination of strain based on strain distribution

The temperature of the neutralized aqueous solution obtained in (1) was set to 25°C, and the frequency of a rheometer (TA Instruments (model: AR-2000ex)) was set to 1 Hz. Subsequently, the storage modulus (G') was measured while applying strain. When a decrease in storage modulus (G') was observed, the measurement was stopped. With reference to the obtained strain dispersion graph, the strain under measurement conditions in (3) below was determined to be 1% when the storage modulus (G') maintained a constant value up to a strain of 1% but failed to maintain a constant value up to 10%, whereas the strain under measurement conditions in (3) below was determined to be 0.1% when the storage modulus (G') maintained a constant value up to a strain of 0.1% but failed to maintain a constant value up to 1%.

(3) Determination of storage modulus based on frequency dispersion

The angular frequency of the neutralized aqueous solution obtained in (1) was changed from 0.1 to 300 rad/s with a strain of 0.1% or 1% based on the strain distribution obtained in (2) at a measurement temperature of 25°C using a rheometer (TA Instruments (model: AR-2000ex)). The storage modulus (G') at an angular frequency of 1 rad/s was determined based on the measurements.

Polymer Production

**Production Example 1**

To a 500 mL (milliliter) four-necked flask equipped with a stirrer, a thermometer, a nitrogen inlet tube, and a condenser were added 40 g of acrylic acid, 0.88 g of Blemmer VMA70 (a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate content; available from NOF Corporation), 0.22 g of pentaerythritol tetraallyl ether, 0.1161 g of 2,2'-azobismethyl isobutyrate, and 230.9 g of n-hexane. The solution was homogeneously mixed by stirring, and then nitrogen gas was blown into the solution to eliminate the oxygen present in the upper space of the reaction vessel (four-necked flask), in the starting materials, and in the solvent. Subsequently, the solution was heated to 60 to 65°C in a nitrogen atmosphere. Then, the temperature was maintained at 60 to 65°C for 3 hours. After maintaining the temperature at 60 to 65°C for about 1 hour, a solution of 0.26 g of polyoxyethylene castor oil (available from Nikko Chemical Co., Ltd.; trade name: CO-3, a 3-mol ethylene oxide adduct) and 0.98 g of polyoxyethylene hydrogenated castor oil triisostearate (available from Nippon Emulsion Co., Ltd.; trade name: RWIS-350, a 50-mol ethylene oxide adduct) in 2.0 g of n-hexane was added to the reaction vessel. Subsequently, the generated slurry was heated to 100°C to distill off n-hexane and further dried under reduced pressure at 115°C at 10 mmHg for 8 hours, thus obtaining 38 g of a water-soluble copolymer having alkyl-modified carboxy groups in a fine white powder form. The copolymer is sometimes referred to as "the polymer of Production Example 1" below.

**Production Example 2**

To a 500 mL four-necked flask equipped with a stirrer, a thermometer, a nitrogen inlet tube, and a condenser were added 45 g (0.625 mol) of acrylic acid, 0.27 g of pentaerythritol tetraallyl ether, 150 g of n-hexane, and 0.153 g (0.000931 mol) of 2,2'-azobismethyl isobutyrate to prepare a reaction solution. The solution was homogeneously mixed by stirring, and then nitrogen gas was blown into the solution to eliminate the oxygen present in the upper space of the reaction vessel, in the starting materials, and in the solvent. Subsequently, the reaction solution was maintained at 60 to 65°C to allow the reaction to proceed for 4 hours in a nitrogen atmosphere. After completion of the reaction, the generated slurry was heated to 90°C to distill off n-hexane and further dried under reduced pressure at 110°C at 10 mmHg for 8 hours, thus obtaining 42 g of a carboxyvinyl polymer. The carboxyvinyl polymer is sometimes referred to as "the polymer of Production Example 2" below.

**Production Example 3**

To a 500 mL four-necked flask equipped with a stirrer, a thermometer, a nitrogen inlet tube, and a condenser were added 45 g (0.625 mol) of acrylic acid, 0.13 g of pentaerythritol tetraallyl ether, 150 g of n-hexane, and 0.153 g (0.000931 mol) of 2,2'-azobismethyl isobutyrate to prepare a reaction solution. The solution was homogeneously mixed by stirring, and then nitrogen gas was blown into the solution to eliminate the oxygen present in the upper space of the reaction vessel, in the starting materials, and in the solvent. Subsequently, the reaction solution was maintained at 60 to 65°C to allow the reaction to proceed for 4 hours in a nitrogen atmosphere. After completion of the reaction, the generated slurry was heated to 90°C to distill off n-hexane and further dried under reduced pressure at 110°C at 10 mmHg for 8 hours, thus obtaining 42 g of a carboxyvinyl polymer. The carboxyvinyl polymer is sometimes referred to as "the polymer of Production Example 3" below.

**Production Example 4**

To a 500 mL four-necked flask equipped with a stirrer, a thermometer, a nitrogen inlet tube, and a condenser were added 45 g (0.625 mol) of acrylic acid, 1.35 g of Blemmer VMA70 (a mixture of 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate; available from NOF Corporation), 0.02 g of pentaerythritol tetraallyl ether, 150 g of n-hexane, and 0.081 g (0.00035 mol) of 2,2'-azobismethyl isobutyrate. The solution was homogeneously mixed by stirring, and then nitrogen gas was blown into the solution to eliminate the oxygen present in the upper space of the reaction vessel (four-necked flask), in the starting materials, and in the solvent. Subsequently, the solution was maintained at 60 to 65°C to allow the reaction to proceed for 4 hours in a nitrogen atmosphere. After completion of the reaction, the generated slurry was heated to 90°C to distill off n-hexane and further dried under reduced pressure at 110°C at 10 mmHg for 8 hours, thus obtaining 43 g of a water-soluble copolymer having alkyl-modified carboxy groups in a fine white powder form. The copolymer is sometimes referred to as "the polymer of Production Example 4" below.

Preparation of Polymer-containing Composition

Viscous compositions having the compositions shown in Tables 1a and 1b were prepared according to the following steps. A BL1200 general-purpose stirrer (available from Shinto Scientific Co., Ltd.) was used as a stirring device, and four paddle blades inclined at 45° were used as stirring blades. The mixing ratio of each component shown in each table is given in parts by mass.

**Example 1**

(1) 53.7 g of ion-exchanged water was added to a 200 mL beaker, and 0.25 g of the polymer of Production Example 1 was added thereto while stirring at 300 rpm. The mixture was stirred at 500 rpm for 30 minutes, thus obtaining a mixed solution (1).
(2) 0.54 g of an 18 mass% aqueous NaOH solution was added to the mixed solution (1) while stirring at 500 rpm. The mixture was stirred at 1000 rpm for 30 minutes, thus obtaining a mixed solution (2). The degree of neutralization of the polymer in the mixed solution was 70%.
(3) 15 g of an amphoteric surfactant (cocamidopropyl betaine SOFTAZOLINE CPB available from Kawaken Fine Chemicals Co., Ltd.) was added to the mixed solution (2) while stirring at 500 rpm. The mixture was stirred for 5 minutes. 30 g of an anionic surfactant (sodium laureth sulfate EMAL 20C available from Kao Corporation) was added to the mixture and stirred for 20 minutes, thus obtaining a mixed solution (3).
(4) 0.5 g of a cationic polymer (polyquaternium-10 CATINAL HC-100 available from Toho Chemical Industry Co., Ltd.) was added to the mixed solution (3) while stirring at 500 rpm, and the mixture was stirred at 1000 rpm for 30 minutes, thus obtaining 100 g of a viscous composition.

**Example 2**

A viscous composition was obtained in the same manner as in Example 1, except that in step 1, the polymer of Production Example 1 was replaced with the polymer of Production Example 2.

**Example 3**

A viscous composition was obtained in the same manner as in Example 1, except that in step 1, the polymer of Production Example 1 was replaced with the polymer of Production Example 3.

**Example 4**

(1) 52.92 g of ion-exchanged water was added to a 200 mL beaker, and 0.50 g of the polymer of Production Example 1 was added thereto while stirring at 300 rpm. The mixture was stirred at 500 rpm for 30 minutes, thus obtaining a mixed solution (1).
(2) 1.08 g of an 18 mass% aqueous NaOH solution was added to the mixed solution (1) while stirring at 500 rpm. The mixture was stirred at 1000 rpm for 30 minutes, thus obtaining a mixed solution (2). The degree of neutralization of the polymer in the mixed solution was 70%.
(3) 15 g of an amphoteric surfactant (cocamidopropyl betaine SOFTAZOLINE CPB available from Kawaken Fine Chemicals Co., Ltd.) was added to the mixed solution (2) while stirring at 500 rpm. The mixture was stirred for 5 minutes. 30 g of an anionic surfactant (sodium laureth sulfate EMAL 20C available from Kao Corporation) was added to the mixture and stirred for 20 minutes, thus obtaining a mixed solution (3).
(4) 0.5 g of a cationic polymer (polyquaternium-10 CATINAL HC-100 available from Toho Chemical Industry Co., Ltd.) was added to the mixed solution (3) while stirring at 500 rpm, and the mixture was stirred at 1000 rpm for 30 minutes, thus obtaining 100 g of a viscous composition.

**Example 5**

(1) 51.34 g of ion-exchanged water was added to a 200 mL beaker, and 1.00 g of the polymer of Production Example 1 was added thereto while stirring at 300 rpm. The mixture was stirred at 500 rpm for 30 minutes, thus obtaining a mixed solution (1).
(2) 2.16 g of an 18 mass% aqueous NaOH solution was added to the mixed solution (1) while stirring at 500 rpm. The mixture was stirred at 1000 rpm for 30 minutes, thus obtaining a mixed solution (2). The degree of neutralization of the polymer in the mixed solution was 70%.
(3) 15 g of an amphoteric surfactant (cocamidopropyl betaine SOFTAZOLINE CPB available from Kawaken Fine Chemicals Co., Ltd.) was added to the mixed solution (2) while stirring at 500 rpm. The mixture was stirred for 5 minutes. 30 g of an anionic surfactant (sodium laureth sulfate EMAL 20C available from Kao Corporation) was added to the mixture and stirred for 20 minutes, thus obtaining a mixed solution (3).
(4) 0.5 g of a cationic polymer (polyquaternium-10 CATINAL HC-100 available from Toho Chemical Industry Co., Ltd.) was added to the mixed solution (3) while stirring at 500 rpm, and the mixture was stirred at 1000 rpm for 30 minutes, thus obtaining 100 g of a viscous composition.

**Comparative Example 1**

(1) 54.5 g of ion-exchanged water was added to a 200 mL beaker, and 15 g of an amphoteric surfactant (cocamidopropyl betaine SOFTAZOLINE CPB available from Kawaken Fine Chemicals Co., Ltd.) was added thereto while stirring at 500 rpm. The mixture was stirred for 5 minutes. 30 g of an anionic surfactant (sodium laureth sulfate EMAL 20C available from Kao Corporation) was added to the mixture and stirred for 20 minutes (a mixed solution (1)).
(2) 0.5 g of a cationic polymer (polyquaternium-10 CATINAL HC-100 available from Toho Chemical Industry Co., Ltd.) was added to the mixed solution (1) while stirring at 500 rpm, and the mixture was stirred at 1000 rpm for 30 minutes, thus obtaining 100 g of a viscous composition.

**Comparative Example 2**

A viscous composition was obtained in the same manner as in Example 1, except that in step 1, the polymer of Production Example 1 was replaced with the polymer of Production Example 4.

**Comparative Example 3**

(1) 54.25 g of ion-exchanged water was added to a 200 mL beaker, and 0.25 g of polyacrylic acid (available from FUJIFILM Wako Pure Chemical Corporation) was added thereto while stirring at 300 rpm. The mixture was stirred at 500 rpm for 30 minutes, thus obtaining a mixed solution (1).

(2) 15 g of an amphoteric surfactant (cocamidopropyl betaine SOFTAZOLINE CPB available from Kawaken Fine Chemicals Co., Ltd.) was added to the mixed solution (1) while stirring at 500 rpm. The mixture was stirred for 5 minutes. 30 g of an anionic surfactant (sodium laureth sulfate EMAL 20C available from Kao Corporation) was added to the mixture and stirred for 20 minutes, thus obtaining a mixed solution (2).

(3) 0.5 g of a cationic polymer (polyquaternium-10 CATINAL HC-100 available from Toho Chemical Industry Co., Ltd.) was added to the mixed solution (2) while stirring at 500 rpm, and the mixture was stirred at 1000 rpm for 30 minutes, thus obtaining 100 g of a viscous composition.

Table 1a

| Component | | | Example | | | | |
|---|---|---|---|---|---|---|---|
| | | | 1 | 2 | 3 | 4 | 5 |
| (a) | Anionic surfactant | Sodium laureth sulfate | 30.00 | 30.00 | 30.00 | 30.00 | 30.00 |
| (b) | Cationic polymer | Polyquatemium-10 | 0.50 | 0.50 | 0.50 | 0.50 | 0.50 |
| (c) | Polymer containing (meth) acrylic acid-derived structural unit | Polymer of Production Example 1 | 0.25 | - | - | 0.50 | 1.00 |
| | | Polymer of Production Example 2 | - | 0.25 | - | - | - |
| | | Polymer of Production Example 3 | - | - | 0.25 | - | - |
| (d) | Water | Ion-exchanged water | 53.71 | 53.71 | 53.71 | 52.92 | 51.34 |
| (e) | Amphoteric surfactant | Cocamidopropyl betaine | 15.00 | 15.00 | 15.00 | 15.00 | 15.00 |
| (f) | Neutralizer | 18% aqueous NaOH solution | 0.54 | 0.54 | 0.54 | 1.08 | 2.16 |
| Properties | Storage modulus (G') at angular frequency of 1 rad/s (Pa) | | 210.5 | 450.3 | 56.9 | 210.5 | 210.5 |

Table 1b

| Component | | | Comparative Example | | |
|---|---|---|---|---|---|
| | | | 1 | 2 | 3 |
| (a) | Anionic surfactant | Sodium laureth sulfate | 30.00 | 30.00 | 30.00 |
| (b) | Cationic polymer | Polyquatemium-10 | 0.50 | 0.50 | 0.50 |
| (c) | Polymer containing (meth)acrylic acid-derived structural unit | Polymer of Production Example 4 | - | 0.25 | - |
| | | Uncrosslinked polyacrylic acid | - | - | 0.25 |
| (d) | Water | Ion-exchanged water | 54.50 | 53.71 | 54.25 |
| (e) | Amphoteric surfactant | Cocamidopropyl betaine | 15.00 | 15.00 | 15.00 |
| (f) | Neutralizer | 18% aqueous NaOH solution | - | 0.54 | - |
| Properties | Storage modulus (G') at angular frequency of 1 rad/s (Pa) | | - | 0.042 | Not measurable |

The physical properties (storage modulus G' (Pa) at 1 rad/s) shown in Tables 1a and 1b above are the results of measuring the storage modulus of each of the polymers containing a (meth)acrylic acid-derived structural unit used in the compositions of the examples and the comparative examples by the measurement method described above (not the results of measuring the storage modulus of each of the compositions of the examples and the comparative examples). Accordingly, since the polymers containing a (meth)acrylic acid-derived structural unit used in Examples 1, 4, and 5 are the same, the values of the physical properties are also the same.

For each of the compositions obtained in the examples and the comparative examples, the pH, viscosity, coacervate formation rate, and coacervate size were measured as follows. Table 2 shows the results. In order to check for the presence or absence of precipitation separation, each composition was shaken uniformly, allowed to stand for 16 hours, and visually inspected. As a result, no precipitation was observed.

pH Measurement

The pH of each viscous composition at a liquid temperature of 25°C was measured using a pH meter (a D-51 glasselectrode hydrogen concentration indicator available from HORIBA, Ltd.).

Viscosity Measurement

The viscosity of each viscous composition was measured at a liquid temperature of 25°C using a BLII Brookfield viscometer (available from Toki Sangyo Co., Ltd.).

Coacervate Formation Rate Calculation

(1) 6 g of the viscous composition and 36 g of ion-exchanged water were added to a 50 mL centrifuge tube and mixed to prepare a 7-fold-diluted viscous composition (total amount: 42 g), which was then left in a constant-temperature bath at 40°C for 16 hours.
(2) Using an H-36 tabletop centrifuge (available from Kokusan Co., Ltd.; rotation radius r: 15 cm), the 7-fold-diluted viscous composition was centrifuged at 3000 rpm for 30 minutes to remove the supernatant so as to collect the residue as coacervates.
(3) The weight of the removed supernatant was measured, and the coacervate formation rate (mass%) was calculated using the following formula.

$$\text{Coacervate formation rate (mass \%)} = \frac{(42 - \text{supernatant (g)})}{42} \times 100$$

Coacervate Size Measurement

(1) 3 g of the aqueous composition and 18 g of ion-exchanged water were added to a 50 mL beaker and mixed to prepare a 7-fold-diluted viscous composition (total amount: 21 g).
(2) The 7-fold-diluted viscous composition was added to a batch cell, the batch cell was set in an SALD-7100 laser diffraction particle size analyzer (available from Shimadzu Corporation) with a stirring plate set in operation and with a refractive index set to 1.60-0.10i, and the median diameter of the particle size distribution measured 50 to 100 seconds after setting the batch cell was determined as the coacervate size. When diluting the composition 7-fold with water, mixing and transferring of the mixture to the batch must be finished within one minute from addition of water to the composition.

Table 2

| | Polymer containing (meth) acrylic acid-derived structural unit | Polymer addition rate | pH | Viscosity (mPa·s) | Coacervate size | | Coacervate formation rate (mass%) |
|---|---|---|---|---|---|---|---|
| | | | | | Elapsed time (s) | Median diameter (μm) | |
| Example 1 | Polymer of Production Example 1 | 0.25 | 7.2 | 95 | 91 | 1.332 | 10.55 |
| Example 2 | Polymer of Production Example 2 | 0.25 | 7.0 | 80 | 55 | 7.579 | 3.17 |
| Example 3 | Polymer of Production Example 3 | 0.25 | 7.1 | 83 | 74 | 8.109 | 2.48 |
| Example 4 | Polymer of Production Example 1 | 0.50 | 6.3 | 780 | 91 | 1.332 | 3.38 |
| Example 5 | Polymer of Production Example 1 | 1.00 | 6.2 | 7940 | 91 | 1.332 | 4.03 |
| Comparative Example 1 | - | - | 7.1 | 33 | 52 | 42.451 | 1.40 |
| Comparative Example 2 | Polymer of Production Example 4 | 0.25 | 7.1 | 123 | 61 | 0.46 | 0.67 |
| Comparative Example 3 | Uncrosslinked polyacrylic acid | 0.25 | 4.6 | 63 | 55 | 51.687 | 1.92 |

Industrial Applicability

The present disclosure provides a novel composition that exhibits excellent coacervate-forming ability. The composition is particularly useful as a hair-washing composition. This makes it possible to produce a hair-washing composition with a high degree of conditioning effect. The composition is also useful for environmental protection because, for example, it can reduce the amount of silicone-based conditioning components used, which are often added to hair-washing compositions.

## Claims

**1.** A composition comprising:

(a) an anionic surfactant, 5 to 50 mass%;
(b) a cationic polymer, 0.025 to 5 mass%;
(c) a polymer containing a (meth)acrylic acid-derived structural unit, 0.1 to 5 mass%; and
(d) water, 20 to 80 mass%,

wherein the polymer (c) is defined as follows:

(A): being at least one polymer selected from the group consisting of (A-1) and (A-2) below:

(A-1) a copolymer obtained by polymerization of the following (i) and (ii); and
(A-2) a copolymer obtained by polymerization of the following (i), (ii), and (iii):

(i) (meth)acrylic acid, 100 parts by mass;
(ii) a compound having two or more ethylenically unsaturated groups, 1 part by mass or less; and
(iii) a (meth)acrylic acid alkyl ester in which the alkyl group has 10 to 30 carbon atoms, 5 parts by mass or less; and

(B): having a storage modulus (G') of 10 to 1000 Pa at a frequency dispersion of 1 rad/s as measured under the

following conditions:
measurement conditions: for a neutralized aqueous solution having a degree of neutralization of 70% and containing 0.5% mass% of the polymer (c) based on the total amount of the neutralized aqueous solution, the storage modulus (G') at an angular frequency of 1 rad/s is determined based on frequency dispersion obtained by changing the angular frequency from 0.1 to 300 rad/s under conditions of a liquid temperature of 25°C and a strain of 0.1% or 1% in strain dispersion with a rheometer frequency set to 1 Hz.

**2.** The composition according to claim 1,

wherein the composition has a coacervate formation rate (mass%) of 2 mass% or more as calculated using the following formula:

$$\text{Coacervate formation rate (mass \%)} = \frac{(42 - \text{supernatant (g)})}{42} \times 100$$

when 42 g of a diluted composition obtained by diluting the composition 7-fold with water is left in a constant-temperature bath at 40°C for 16 hours and centrifuged at 3000 rpm for 30 minutes to remove the supernatant so as to collect the residue as coacervates.

**3.** The composition according to claim 1 or 2,
wherein the composition has a coacervate size of 0.1 to 30 μm when the coacervate size is determined as follows: a diluted composition obtained by diluting the composition 7-fold with water is added to a batch cell within one minute from addition of water to the composition for dilution, the batch cell is set in a laser diffraction particle size analyzer with a stirring plate set in operation and with a refractive index set to 1.60-0.10i, and the median diameter of the particle size distribution measured 50 to 100 seconds after setting the batch cell is determined as the coacervate size.

**4.** The composition according to claim 1 or 2,
wherein the anionic surfactant (a) is at least one selected from the group consisting of lauryl sulfate, laureth sulfate, lauryl monoglyceride sulfate, lauryl sarcosine or a salt thereof, lauroyl sarcosine or a salt thereof, cocoyl sulfate, cocoyl glutamate, cocoyl glycinate salt, cocoyl methylalaninate salt, cocoyl aspartate, cocoyl sarcosinate salt, tridecylbenzenesulfonate, dodecylbenzenesulfonic acid or a salt thereof, and cocoyl isethionic acid or a salt thereof.

**5.** The composition according to claim 1 or 2,

wherein the cationic polymer (b) is at least one selected from the group consisting of
a copolymer of methylvinylimidazolinium chloride and vinylpyrrolidone,
a copolymer of 1-vinyl-2-pyrrolidone and dimethylaminoethyl methacrylate,
a polymer of dimethyldiallylammonium chloride,
a copolymer of dimethyldiallylammonium chloride and acrylamide,
a copolymer of dimethyldiallylammonium chloride and acrylic acid,
a copolymer of acrylic acid, diallyldimethylammonium chloride, and acrylamide,
a copolymer of acrylic acid, methyl acrylate, and methacrylamidopropyltrimethylammonium chloride,
a copolymer of acrylamide, dimethyldiallylammonium chloride, and methacrylamidopropyltrimonium chloride,
a polymer obtained by addition of glycidyltrimethylammonium chloride to hydroxyethyl cellulose, and
a polymer obtained by addition polymerization of glycidyllauryldimethylammonium chloride to hydroxyethyl cellulose.

**6.** The composition according to claim 1 or 2, which is for washing hair.

**7.** A method for washing hair using the hair-washing composition according to claim 6.

**8.** A method for washing hair, comprising:

applying the hair-washing composition according to claim 6 to hair; and
rinsing the applied hair-washing composition with water.

**9.** A method for improving the yield of coacervates by using (c) defined below, the coacervates being generated when a

composition comprising the following (a) to (d) is diluted with water:

(a) an anionic surfactant, 5 to 50 mass%;
(b) a cationic polymer, 0.025 to 5 mass%;
(c) a polymer containing a (meth)acrylic acid-derived structural unit, 0.1 to 5 mass%; and
(d) water, 20 to 80 mass%,

wherein the polymer (c) is defined as follows:

(A): being at least one polymer selected from the group consisting of (A-1) and (A-2) below:

(A-1) a copolymer obtained by polymerization of the following (i) and (ii); and
(A-2) a copolymer obtained by polymerization of the following (i), (ii), and (iii):

(i) (meth)acrylic acid, 100 parts by mass;
(ii) a compound having two or more ethylenically unsaturated groups, 1 part by mass or less; and
(iii) a (meth)acrylic acid alkyl ester in which the alkyl group has 10 to 30 carbon atoms, 5 parts by mass or less, and

(B): having a storage modulus (G') of 10 to 1000 Pa at a frequency dispersion of 1 rad/s as measured under the following conditions:
measurement conditions: for a neutralized aqueous solution having a degree of neutralization of 70% and containing 0.5% mass% of the polymer (c) based on the total amount of the neutralized aqueous solution, the storage modulus (G') at an angular frequency of 1 rad/s is determined based on frequency dispersion obtained by changing the angular frequency from 0.1 to 300 rad/s under conditions of a liquid temperature of 25°C and a strain of 0.1% or 1% in strain dispersion with a rheometer frequency set to 1 Hz.

**10.** The method for improving the yield of coacervates according to claim 9,

wherein the coacervate formation rate (mass%) is 2 mass% or more as calculated using the following formula:

$$\text{Coacervate formation rate (mass \%)} = \frac{(42 - \text{supernatant (g)})}{42} \times 100$$

when 42 g of a diluted composition obtained by diluting the composition comprising (a) to (d) 7-fold with water is left in a constant-temperature bath at 40°C for 16 hours and centrifuged at 3000 rpm for 30 minutes to remove the supernatant so as to collect the residue as coacervates.

**11.** A method for improving the yield of coacervates, comprising diluting the composition according to claim 1 or 2 with water to generate coacervates,
wherein the yield of coacervates is improved as compared to when coacervates are generated by diluting with water a composition comprising the components (a) to (d), the component (c) being 0.1 to 5 mass% of a polymer containing a (meth)acrylic acid-derived structural unit that does not satisfy the condition (B).

Fig. 1

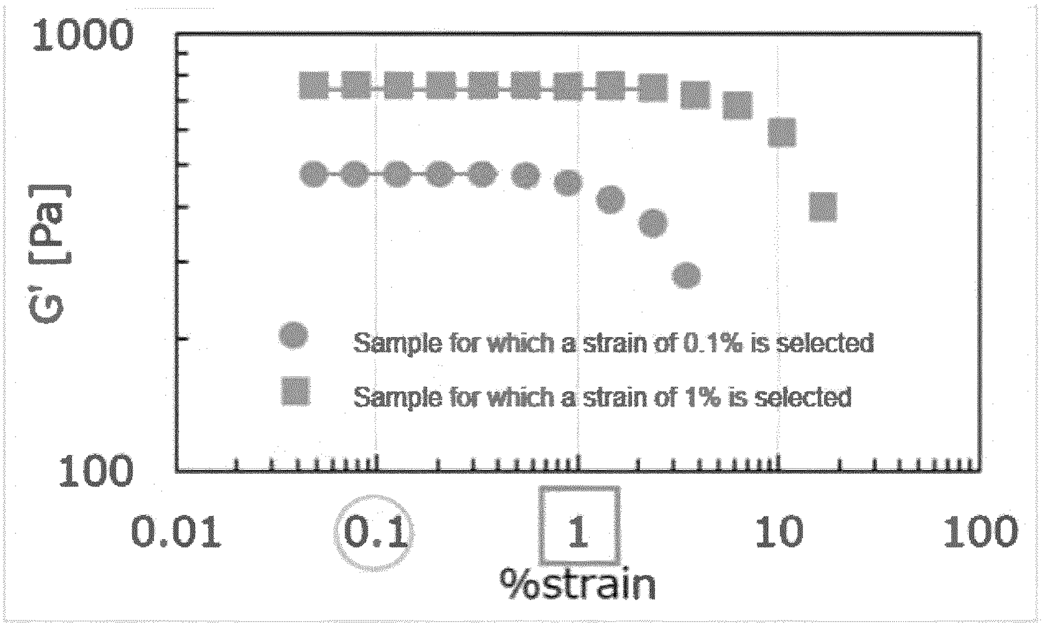
1000
100
G' [Pa]
Sample for which a strain of 0.1% is selected
Sample for which a strain of 1% is selected
0.01
0.1
1
10
100
%strain

Fig. 2

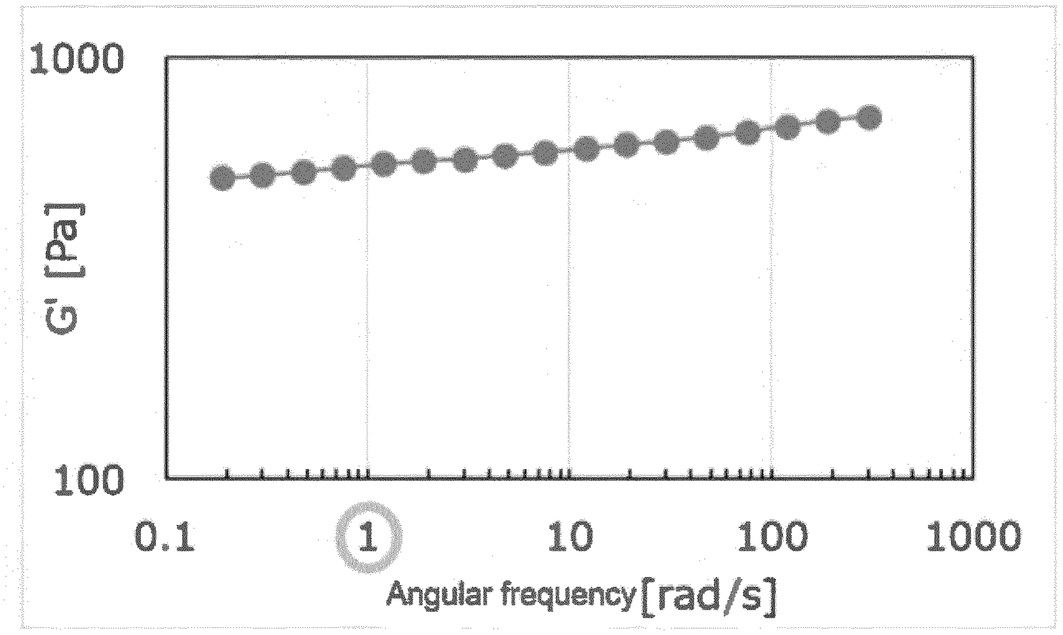
1000
100
G' [Pa]
0.1
1
10
100
1000
Angular frequency [rad/s]

**INTERNATIONAL SEARCH REPORT**

International application No. **PCT/JP2024/010737**

**A. CLASSIFICATION OF SUBJECT MATTER**

***A61K 8/81***(2006.01)i; ***A61K 8/44***(2006.01)i; ***A61K 8/46***(2006.01)i; ***A61K 8/73***(2006.01)i; ***A61Q 5/02***(2006.01)i
FI: A61K8/81; A61Q5/02; A61K8/46; A61K8/73; A61K8/44

According to International Patent Classification (IPC) or to both national classification and IPC

**B. FIELDS SEARCHED**

Minimum documentation searched (classification system followed by classification symbols)

A61K8/81; A61K8/44; A61K8/46; A61K8/73; A61Q5/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS/KOSMET (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2020/179092 A1 (SUMITOMO SEIKA CHEMICALS CO., LTD.) 10 September 2020 (2020-09-10)<br>entire text | 1-11 |
| A | WO 2020/262043 A1 (SUMITOMO SEIKA CHEMICALS CO., LTD.) 30 December 2020 (2020-12-30)<br>entire text | 1-11 |
| A | JP 2010-202627 A (SUMITOMO SEIKA CHEMICALS CO., LTD.) 16 September 2010 (2010-09-16)<br>entire text | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 April 2024** | **14 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/010737**

| Patent document cited in search report | Publication date (day/month/year) | Patent family member(s) | Publication date (day/month/year) |
|---|---|---|---|
| WO 2020/179092 A1 | 10 September 2020 | US 2022/0056256 A1<br>entire text<br>EP 3932988 A1<br>CN 113508158 A<br>KR 10-2021-0134907 A | |
| WO 2020/262043 A1 | 30 December 2020 | US 2022/0251365 A1<br>entire text<br>EP 3991799 A1<br>CN 114080216 A<br>KR 10-2022-0026577 A | |
| JP 2010-202627 A | 16 September 2010 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008129493 A
- JP 2015224197 A
- WO 2003094875 A